# EUROPEAN PATENT SPECIFICATION

(11) **EP 2 640 683 B1**
(45) Date of publication and mention of the grant of the patent: **13.08.2014**
(21) Application number: 11779100.4
(22) Date of filing: 27.10.2011
(51) Int. Cl.: C07C 41/01, C07C 43/04, C10K 3/00, C10K 3/02, C10L 3/08

(54) **PROCESS FOR THE PREPARATION OF GASEOUS SYNFUEL**
VERFAHREN ZUR HERSTELLUNG VON GASFÖRMIGEM SYNTHETISCHEM HEIZÖL
PROCÉDÉ DE PRÉPARATION DE COMBUSTIBLE ARTIFICIEL GAZEUX

(30) Priority: 17.11.2010 DK 201001042
(43) Date of publication of application: 25.09.2013
(73) Proprietor: Haldor Topsøe A/S, 2800 Kgs. Lyngby (DK)
(72) Inventor: NIELSEN, Poul Erik Højlund, DK-3480 Fredensborg (DK); MADSEN, Jørgen, DK-3400 Hillerød (DK)
(86) International application number: PCT/EP2011/005417
(87) International publication number: WO 2012/065684

(56) References cited:
- EP-A1- 2 028 173
- EP-A1- 2 070 905
- DE-A1- 4 222 655
- LARSON E D ET AL: "Dimethyl ether (DME) from coal as a household cooking fuel in China", ENERGY SUSTAINABLE DEVELOPMENT, INTERNATIONAL ENERGY INITIATIVE, BANGALORE, IN, vol. 8, no. 3, 1 September 2004 (2004-09-01), pages 115-126, XP025847853, ISSN: 0973-0826, DOI: 10.1016/S0973-0826(08)60473-1 [retrieved on 2004-09-01]
- MARCHIONNA M ET AL: "Fundamental investigations on di-methyl ether (DME) as LPG substitute or make-up for domestic uses", FUEL PROCESSING TECHNOLOGY, ELSEVIER BV, NL, vol. 89, no. 12, 1 December 2008 (2008-12-01), pages 1255-1261, XP025681086, ISSN: 0378-3820, DOI: 10.1016/J.FUPROC.2008.07.013 [retrieved on 2008-08-28]
- JU F ET AL: "Process simulation of single-step dimethyl ether production via biomass gasification", BIOTECHNOLOGY ADVANCES, ELSEVIER PUBLISHING, BARKING, GB, vol. 27, no. 5, 1 September 2009 (2009-09-01), pages 599-605, XP026152509, ISSN: 0734-9750, DOI: 10.1016/J.BIOTECHADV.2009.04.015 [retrieved on 2009-04-23]

## Description

The present invention is related to a process for the preparation of a gaseous synfuel having a heating value in the same range as natural gas.

The invention is in particular useful when a carbonaceous feedstock is available and there is a need for producing a gaseous synfuel capable of replacing natural gas.

By conventional technology carbonaceous feed stock is gasified with oxygen in order to obtain a basically inert gasfree synthesis gas, which in several catalytic steps can be converted into substitute natural gas (SNG).

For use in smaller plants, oxygen is too expensive, and air-blown gasification is typically employed in these plants.

When gaseous SNG is produced by air-blown gasification, the problem arises that the SNG product, i.e. mainly methane, are introduced into the SNG product, said product containing large amounts of nitrogen having been contained in the air. Separation of CH4 from such a mixture is costintensive.

A mixture of nitrogen and dimethyl ether (DME) appears to be inter-changeable with base natural gas. Thus, a mixture of for instance 40% by volume of N₂ and 60% by volume of DME has the same fuel value (BTU) as natural gas.

The invention is based upon the above fact as well as on the finding that a high content of nitrogen in the synthesis gas mixture does not adversely effect a subsequent catalytic conversion of the gas to dimethyl ether. Furthermore, nitrogen and other inerts are much easier removed from DME than from SNG in order to obtain the desired BTU. As an advantage of the above findings, synthesis gas for the preparation of DME based synfuel for the replacement of natural gas may contain nitrogen and is manufacturable by less expensive methods comparable to that of synthesis gas for preparation of SNG.

Thus, a general embodiment of the invention is a process for the preparation of a gaseous dimethyl ether synfuel having a BTU value corresponding substantially to the BTU value of natural gas, said process comprising the steps of providing a synthesis gas comprising hydrogen, carbon monoxide and nitrogen;
catalytically converting the synthesis gas into a dimethyl ether raw product further comprising unconverted synthesis gas, the nitrogen and dissolved carbon dioxide;
cooling and condensing the dimethyl ether raw product with the dissolved carbon dioxide to a liquid and a gaseous phase with the unconverted synthesis gas and the nitrogen; separating the liquid phase from the gaseous phase; treating the liquid phase to remove the carbon dioxide from the dimethyl ether raw product and to obtain a purified dimethyl ether product; and
admixing and adjusting content of inert gas having a BTU value of about zero into the purified dimethyl ether raw product to obtain the gaseous dimethyl ether synfuel having a BTU value substantially equal to that of the BTU value of natural gas, wherein the synthesis gas is prepared by air-blown gasification of a carbonaceous material.

The carbonaceous material can be any solid material containing carbon, including coal and biomass.

The important carbon gasification reactions are

C + O₂ → CO₂

C + CO₂ → 2CO

C + H₂O → CO + H₂

CO + H₂O → CO₂ + H₂

The carbonaceous material is preferably gasified with air at a pressure of between 2 to 3 MPa, which is the typically employed maximum pressure at which the solid carbonaceous material is fed into the gasifier.

The gasification temperature is typically between 600°C and 900°C and below the ash agglomeration point.

In addition to carbon oxides and hydrogen, a raw synthesis gas obtained by air-blown gasification comprises tar and more than 30 mole % of inert gases, mainly nitrogen, and methane.

The content of methane is preferably removed from the raw gas by tubular steam reforming of the gas in accordance with the conventional steam reforming processes, wherein methane is steam reformed to hydrogen and carbon oxides.

Synthesis gas prepared by coal or biomass gasification has additionally a relatively high content of dust and tar when it is gasified in a fluid bed gasifier. Tar is formed in the gasifier and comprises a wide spectrum of organic compounds, which should be removed from the raw gas prior to conversion of the synthesis gas into DME in order to avoid fouling of the process equipment and a plugging and poisoning of the catalyst in the DME reactor.

Accordingly it is preferred to remove the dust in the raw synthesis gas from the gasifier by conventional means and to remove the tar by adiabatic steam reforming into methane, hydrogen and carbon oxides.

The reforming of tar is accomplished at temperatures from 650°C to 900°C in contact with a modified steam methane reforming catalyst known in the art, such as Ni on different supports, including Al₂O₃, ZrO₂, TiO₂, SiO₂, or a Ni/MgO-CaO catalyst, or M-CeO₂-SiO₂, where M = Rh, Pt, Pd, Ru, Ni.

The raw gas from the gasifier contains in addition certain impurities, which have a poisonous effect on downstream catalysts being employed in the subsequent conversion of the tar and of the synthesis gas into DME. These impurities are particularly carbonyl sulphide, metal carbonyls, carbon disulphide, hydrogen sulphide, hydrogen cyanide, ammonia and arsenic and chlorine.

Thus, according to a further embodiment of the invention, the carbonyl sulphide, metal carbonyls, carbon disulphide, hydrogen sulphide, hydrogen cyanide, ammonia and arsenic and chlorine are removed from the raw gas by contacting the gas with a series of sorbents having a sufficient sorption activity for adsorption or absorption of the above mentioned impurities. These sorbents and the use thereof are described in European patent application no. 2 156 877 A, in which the raw gas is contacted in succession with a first purification agent comprising active carbon, with a second purification agent comprising alumina, with a third purification agent comprising zinc oxide, with a fourth purification agent comprising a zeolitic material and a fifth purification agent comprising zinc oxide and copper oxide.

The conversion of the nitrogen containing synthesis gas into dimethyl ether is carried out in one or more reactors in which the synthesis gas is catalytically converted into methanol, cf. equation (1), and dimethyl ether as shown in equation (2). The shift reaction also takes place and is shown in equation (3).

CO + 2H₂ → CH₃OH (1)

2CH₃OH → CH₃OCH₃ + H₂O (2)

CO + H₂O → CO₂ + H₂ (3)

The catalysts active in the conversion of synthesis gas into methanol and dimethyl ether are well known in the art.

Maximum conversion of synthesis gas is obtained when dimethyl ether is prepared at a stoichiometric ratio between hydrogen and carbon monoxide equal to one. At ratios above or below this ratio one less dimethyl ether is prepared. At maximum conversion (H₂/CO ≈ 1), the overall reaction takes place essentially according to equation (4):

3H₂ + 3CO → CH₃OCH₃ + CO₂ (4)

The carbon dioxide present in the synthesis gas and formed during the above reaction (3) is soluble in dimethyl ether. To obtain the dimethyl ether product with a required BTU value, it is necessary to remove the carbon dioxide.

In accordance with an embodiment of the invention, the effluent from the dimethyl ether synthesis is cooled, and the dimethyl ether raw product containing dissolved carbon dioxide, unconverted synthesis gas, methane and nitrogen is cooled and condensed to the liquid phase. The remaining gas phase containing the above-mentioned gaseous components is separated from the liquid phase to obtain partially purified dimethyl ether with carbon dioxide dissolved in the ether.

The separated dimethyl ether raw product is preferably washed in a scrubbing zone with a liquid solvent rich in potassium carbonate or amine. Thereby, the carbon dioxide is selectively absorbed in the liquid solvent.

The liquid solvent for the removal of carbon dioxide contains typically 20 to 40 wt% potassium carbonate.

The gaseous dimethyl ether synfuel prepared according to the invention can be used as pipeline gas in an existing natural gas distribution net.

In order to be useful as a gaseous synfuel for the replacement of natural gas or for mixing with natural gas, the BTU value of the purified dimethyl ether raw product must be adjusted by admixing inert compounds having a BTU of about zero into the product to adapt the BTU value to that of the natural gas.

Nitrogen and other inert diluents are suitable for use in the admixture into the purified dimethyl ether product and the adjustment of the BTU value of the product.

Nitrogen is easily available from many sources. Nitrogen gas is for instance produced by fractional distillation of liquid air, or by mechanical means using gaseous air and pressurized reverse osmosis or pressure swing adsorption.

## Claims

1. Process for the preparation of a gaseous dimethyl ether synfuel having a BTU value corresponding substantially to the BTU value of natural gas, said process comprising the steps of
providing a synthesis gas comprising hydrogen, carbon monoxide and nitrogen;
catalytically converting the synthesis gas into a dimethyl ether raw product further comprising unconverted synthesis gas, the nitrogen and dissolved carbon dioxide;
cooling and condensing the dimethyl ether raw product with the dissolved carbon dioxide to a liquid and a gaseous phase with the unconverted synthesis gas and the nitrogen; separating the liquid phase from the gaseous phase; treating the liquid phase to remove the carbon dioxide from the dimethyl ether raw product and to obtain a purified dimethyl ether product; and
admixing and adjusting content of inert gas having a BTU value of about zero into the purified dimethyl ether raw product to obtain the gaseous dimethyl ether synfuel having a BTU value equal to that of the BTU value of natural gas, wherein the synthesis gas is prepared by air-blown gasification of a carbonaceous material.

2. The process of claim 1, wherein the feedstock is gasified with air at a pressure of between 2 to 3 MPa.

3. The process of claim 1 or 2, comprising the further steps of removal of the dust from the synthesis gas and steam reforming of tar contained in the synthesis gas from the air-blown gasification.

4. The process according to anyone of claim 1 to 3, wherein the synthesis gas is further steam reformed upstream the catalytically converting of the synthesis gas into dimethyl ether raw product.

5. The process according to anyone of claim 1 to 4, wherein the synthesis gas is pressurized to a pressure of between 8 to 10 MPa prior to the catalytically conversion into the dimethyl ether raw product.

6. The process according to anyone of claim 1 to 5, wherein the liquid phase is treated with a liquid solvent of an amine or a liquid solvent containing potassium carbonate for the removal of carbon dioxide from the dimethyl ether raw product.

7. The process according to anyone of claim 1 to 6, comprising the further step of removing carbonyl sulphide, metal carbonyls, carbon disulphide, hydrogen sulphide, hydrogen cyanide, ammonia and arsenic and chlorine from the synthesis gas.

8. Use of the gaseous dimethyl ether synfuel prepared according to anyone of the preceding claims as pipeline gas in an existing natural gas distribution net.

## Patentansprüche

1. Verfahren zur Herstellung eines synthetischen gasförmigen Dimethylether Treibstoffs, der einen im wesentlichen dem BTU-Wert von Erdgas entsprechenden BTU-Wert aufweist, wobei das Verfahren die Schritte umfasst:
Bereitstellen eines Synthesegases umfassend Wasserstoff, Kohlenmonoxid und Stickstoff;
katalytische Umwandlung des Synthesegases in ein Dimethylether-Rohprodukt weiter umfassend nicht umgewandeltes Synthesegas, den Stickstoff und gelöstes Kohlendioxid;
Kühlen und Kondensieren des Dimethylether-Rohprodukts mit dem gelösten Kohlendioxid zu einer flüssigen und einer gasförmigen Phase mit dem nicht umgesetzten Synthesegas und dem Stickstoff;
Abtrennen der flüssigen Phase von der gasförmigen Phase;
Behandeln der flüssigen Phase, um das Kohlendioxid aus dem Dimethylether-Rohprodukt zu entfernen und um ein gereinigtes Dimethylether-Produkt zu erhalten; und
Zumischen und Anpassen des Gehalts von Inertgas mit einem BTU-Wert von ungefähr Null zu dem gereinigten Dimethylether-Rohprodukt, um den synthetischen gasförmigen Dimethylether Treibstoff, der einen im wesentlichen dem BTU-Wert von Erdgas entsprechenden BTU-Wert aufweist, zu erhalten, bei dem das Synthesegas durch eine mit Luft betriebene Vergasung eines kohlenstoffhaltigen Materials hergestellt wird.

2. Verfahren nach Anspruch 1, wobei das Ausgangsmaterial mit Luft bei einem Druck von 2 bis 3 MPa vergast wird.

3. Verfahren gemäß Anspruch 1 oder 2, umfassend die weiteren Schritte der Entfernung von Staub aus dem Synthesegas und der Dampfreformierung von Teer, der in dem Synthesegas aus der mit Luft betriebenen Vergasung enthalten ist.

4. Verfahren gemäß irgendeinem der Ansprüche 1 bis 3, wobei das Synthesegas ferner stromaufwärts der katalytischen Umwandlung von Synthesegas zu Dimethylether-Rohprodukt dampfreformiert wird.

5. Verfahren gemäß irgendeinem der Ansprüche 1 bis 4, wobei das Synthesegas vor der katalytischen Umwandlung in das Dimethylether-Rohprodukt auf einen Druck von 8 bis 10 MPa vorgespannt wird.

6. Verfahren gemäß irgendeinem der Ansprüche 1 bis 5, wobei die flüssige Phase zur Entfernung von Kohlendioxid aus dem Dimethylether-Rohprodukt mit einem flüssigen Lösungsmittel aus einem Amin oder einem flüssigen Lösungsmittel enthaltend Kaliumcarbonat behandelt wird.

7. Verfahren gemäß irgendeinem der Ansprüche 1 bis 6, umfassend den weiteren Schritt des Entfernens von Carbonylsulfid, Metallcarbonylen, Schwefelkohlenstoff, Schwefelwasserstoff, Cyanwasserstoff, Ammoniak und Arsen und Chlor aus dem Synthesegas.

8. Verwendung des synthetischen gasförmigen Dimethylether Treibstoffs gemäß irgendeinem der vorhergehenden Ansprüche als Ferngas in einem vorhandenen Erdgasverteilungsnetz.

## Revendications

1. Procédé de préparation d'un combustible artificiel diméthyléther gazeux ayant une valeur BTU correspondant substantiellement à la valeur BTU du gaz naturel, ledit procédé comprenant les étapes consistant à
se procurer un gaz de synthèse comprenant de l'hydrogène, du monoxyde de carbone et de l'azote ;
convertir par catalyse le gaz de synthèse en un produit brut diméthyléther comprenant encore du gaz de synthèse non converti, l'azote et du dioxyde de carbone dissous ;
refroidir et condenser le produit brut diméthyléther avec le dioxyde de carbone dissous pour donner une phase liquide et une phase gazeuse avec le gaz de synthèse non converti et l'azote ;
séparer la phase liquide de la phase gazeuse ;
traiter la phase liquide pour éliminer le dioxyde de carbone du produit brut diméthyléther et pour obtenir un produit diméthyléther purifié ; et
mélanger et ajuster la teneur en gaz inerte ayant une valeur BTU d'environ zéro dans le produit brut diméthyléther purifié pour obtenir le combustible artificiel diméthyléther gazeux ayant une valeur BTU égale à la valeur BTU du gaz naturel, le gaz de synthèse étant préparé par gazéification d'une matière carbonée par insufflation d'air.

2. Procédé de la revendication 1, dans lequel la charge d'alimentation est gazéifiée avec de l'air à une pression comprise entre 2 et 3 MPa.

3. Procédé de la revendication 1 ou 2, comportant les étapes supplémentaires d'élimination des poussières du gaz de synthèse et de reformage à la vapeur des goudrons contenus dans le gaz de synthèse résultant de la gazéification par insufflation d'air.

4. Procédé selon l'une quelconque des revendications 1 à 3, dans lequel le gaz de synthèse est en outre reformé à la vapeur en amont de la conversion catalytique du gaz de synthèse en produit brut diméthyléther.

5. Procédé selon l'une quelconque des revendications 1 à 4, dans lequel le gaz de synthèse est pressurisé à une pression comprise entre 8 et 10 MPa avant la conversion catalytique en produit brut diméthyléther.

6. Procédé selon l'une quelconque des revendications 1 à 5, dans lequel la phase liquide est traitée avec un solvant liquide d'une amine ou un solvant liquide contenant du carbonate de potassium pour l'élimination du dioxyde de carbone du produit brut diméthyléther.

7. Procédé selon l'une quelconque des revendications 1 à 6, comportant l'étape supplémentaire d'élimination du sulfure de carbonyle, des carbonyles métalliques, du disulfure de carbone, du sulfure d'hydrogène, du cyanure d'hydrogène, de l'ammoniac et de l'arsenic et du chlore, du gaz de synthèse.

8. Utilisation du combustible artificiel diméthyléther gazeux préparé selon l'une quelconque des revendications précédentes comme gaz de pipeline dans un réseau de distribution de gaz naturel existant.
